# EUROPÄISCHE PATENTSCHRIFT

(11) **EP 1 893 096 B1**
(45) Veröffentlichungstag und Bekanntmachung des Hinweises auf die Patenterteilung: **26.12.2018**
(21) Anmeldenummer: 06721197.9
(22) Anmeldetag: 06.04.2006
(51) Int. Cl.: A61B 8/12, A61B 8/08, A61B 1/31, A61B 17/00

(54) **EINRICHTUNG ZUR VERWENDUNG BEI DER LIGATUR VON INTRAMURALEN ARTERIEN**
DEVICE USED FOR INTRAMURAL ARTERIAL LIGATURES
DISPOSITIF UTILISABLE POUR DES LIGATURES D'ARTERES INTRAMURALES

(30) Priorität: 04.05.2005 AT 29405 U
(43) Veröffentlichungstag der Anmeldung: 05.03.2008
(73) Patentinhaber: A.M.I. Agency for Medical Innovations GmbH, 6800 Feldkirch (AT)
(72) Erfinder: EGLE, Walter, 6842 Koblach (AT)
(74) Vertreter: Hofmann, Ralf U.
(86) Internationale Anmeldenummer: PCT/AT2006/000139
(87) Internationale Veröffentlichungsnummer: WO 2006/116779

(56) Entgegenhaltungen:
- EP-A- 1 234 539
- US-A1- 2002 143 275
- US-A1- 2004 024 302
- US-A1- 2004 181 154

## Beschreibung

Die Erfindung betrifft eine Einrichtung zur Verwendung bei der Ligatur von intramuralen Arterien in Hohlorganen, mit einem mindestens einen Handgriff aufweisenden Basisteil und einem am Basisteil angebrachten, in das Hohlorgan, insbesondere das Rektum, einzuführenden Tubus, an dem zur Ortung der zu legierenden Arterien eine Ultraschallsonde angeordnet ist, die mit einer Ansteuer- und Auswertelektronik verbunden ist, welche Ein- und Ausgabeelemente umfasst.

Derartige, auch als Ultraschall-Proktoskope bezeichnete Einrichtungen sind beispielsweise aus der US 5,570,692 A und EP 1 234 539 A1 bekannt. Der Tubus besitzt in seinem distalen Bereich eine Behandlungsöffnung, die von einer Öffnung in der Seitenwand des Tubus oder auch vom distalen offenen Ende des Tubus gebildet wird. Durch das offene proximale Ende des in den Anus eingeführten Tubus kann die Behandlungsöffnung eingesehen werden, durch welche eine Ligatur der intramuralen Arterien erfolgt. Zur Lokalisation einer jeweiligen intramuralen Arterie ist am Tubus eine Ultraschallsonde angeordnet, die mit einer Elektronikeinheit verbunden ist. Das von der Ultraschallsonde empfangene reflektierte Signal wird von der Auswertelektronik der Elektronikeinheit ausgewertet und in ein akustisches Signal umgesetzt. Daneben kann als Ausgabeelement auch eine optische Anzeige vorhanden sein, von der angezeigt wird, wenn sich die Ultraschallsonde über einer Arterie befindet.

Das Handgerät, welches ein einen Handgriff aufweisendes Basisteil und den am Basisteil angebrachten, mit der Ultraschallsonde versehenen Tubus umfasst, ist über ein Anschlusskabel mit der Elektronikeinheit verbunden, die die Ansteuer- und Auswertelektronik für die Ultraschallsonde mit Ein- und Ausgabeelementen umfasst. Die Stromversorgung erfolgt über ein Netzteil. Zusätzlich können in die Elektronikeinheit eingesetzte Batterien zur Notstromversorgung vorhanden sein. Als Ausgabeelement ist beispielsweise in der Elektronikeinheit ein Lautsprecher angeordnet, als Eingabeelement ein Lautstärkeregler. Daneben können auch Ein- und Ausgabeelemente am Basisteil angeordnet sein.

Nachteilig an diesen herkömmlichen Einrichtungen ist es, dass das Anschlusskabel zwischen dem Handgerät und der Elektronikeinheit ein Sterilitätsrisiko darstellt. Insbesondere aufgrund der bei der Operation erforderlichen Drehungen des Tubus, um die zu ligierenden intramuralen Arterien aufzufinden, kann das Anschlusskabel, das vor der Operation sterilisiert wird, bei der Operation leicht mit unsterilen Flächen in Berührung kommen. Dadurch geht seine Sterilität verloren, was eine Gefahr darstellt, wenn es in der Folge wiederum mit sterilen Flächen in Berührung kommt. Weiters beeinträchtigt diese Anschlusskabel die leichte Hantierbarkeit des Handgeräts. Bei der Operation muss die Sonde sehr feinfühlig verschoben und gedreht werden, um die zu ligierenden Arterien exakt zu lokalisieren.

Weitere Ultraschallgeräte zur medizinischen Verwendung sind aus der US 2004/181154 A1, US 2004/024302 A1 und US 2002/143275 A1 bekannt, wobei aus letztgenannter Schrift auch ein autarkes Handgerät hervorgeht.

Aufgabe der Erfindung ist es, eine verbesserte Einrichtung der eingangs genannten Art bereitzustellen, durch die die erforderliche Sterilität besser gewährleistet ist und die eine bessere Handhabung zur einfacheren Ausrichtung des Tubus und Lokalisierung der zu ligierenden Arterien ermöglicht. Erfindungsgemäß gelingt dies durch eine Einrichtung mit den Merkmalen des Anspruchs 1.

Durch die Erfindung entfällt ein Verbindungskabel zwischen dem Basisteil und einer Elektronikeinheit, d. h. die Einrichtung ist kabellos. Die Einrichtung ist insgesamt als autarkes Handgerät ausgebildet, d. h. zumindest bei der Operation umfasst die Einrichtung keine weiteren Teile als dieses Handgerät.

In einer vorteilhaften Ausführungsform der Erfindung bilden Außenwände des Basisteils ein Gehäuse für die Ansteuer- und Auswertelektronik und den Energiespeicher. Die mit der Ultraschallsonde verbundene Elektronikeinheit stellt in diesem Sinn einen integralen Bestandteil des Basisteils dar.

Gemäß der Erfindung weist das Basisteil ein Gehäuse auf, innerhalb dessen eine mit einem eigenen Gehäuse versehene Elektronikeinheit angeordnet ist, welche die Ansteuer- und Auswertelektronik für die Ultraschallsonde mit Ein- und Ausgabeelementen und einen Energiespeicher umfasst. Das Gehäuse des Basisteils ist hierbei öffenbar, wobei im geöffneten Zustand des Gehäuses die Elektronikeinheit aus dem Basisteil herausnehmbar bzw. in dieses einsetzbar ist. Zum Sterilisieren des Basisteils kann somit die Elektronikeinheit aus diesem herausgenommen werden.

Weitere Vorteile und Einzelheiten der Erfindung werden im Folgenden anhand der beiliegenden Zeichnung erläutert. In dieser zeigen:
- Fig. 1: eine nicht unter die Erfindung fallende Ausführungsform in einer Schrägsicht von schräg vorne;
- Fig. 2: die Einrichtung von Fig. 1 in einer Schrägsicht von schräg hinten;
- Fig. 3: eine Schrägsicht der Einrichtung mit abgenommenem Tubus;
- Fig. 4: eine Schrägsicht der Einrichtung, wobei der Tubus abgenommen ist und der hintere Gehäusedeckel geöffnet ist;
- Fig. 5: eine Ausführungsform der Erfindung, in einer Schrägsicht von schräg hinten;
- Fig. 6: die Einrichtung von Fig. 5 mit geöffnetem Basisteil und herausgenommener Elektronikeinheit;
- Fig. 7: die Einrichtung im Zustand von Fig. 6 in einer Schrägsicht aus einem anderen Blickwinkel.

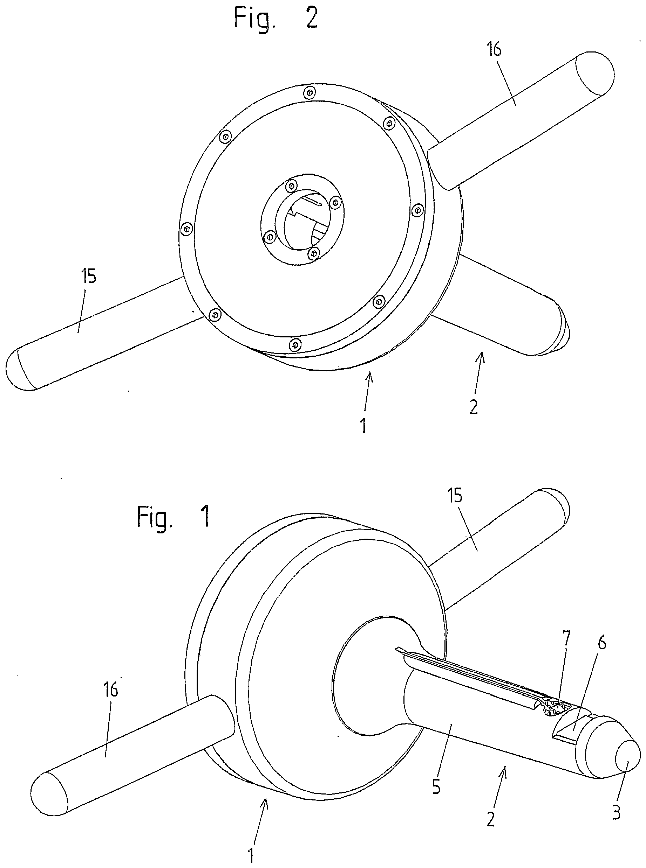

Ein nicht unter die Erfindung fallendes Ausführungsbeispiel ist in den Fig. 1 bis 4 dargestellt. Die Einrichtung umfasst ein Basisteil 1 und einen am Basisteil 1 abnehmbar befestigten Tubus 2 (= ein hülsenartiges Teil). Der bei der Operation in den Anus einzuführende Tubus 2 besitzt einen inneren Hohlraum, der zum distalen Ende 3 des Tubus 2 hin geschlossen ist und am proximalen Ende 4 des Tubus 2 offen ist. Umfänglich ist der innere Hohlraum von einer Mantelwand 5 umgeben, distal von einem kappenartigen Teil geschlossen. In der Mantelwand 5 des Tubus 2 ist eine zur Seite gerichtete Behandlungsöffnung 6 ausgebildet, durch die bei der Operation die Mukosa zugänglich ist, um intramurale Arterien zu ligieren.

Zur Ortung der zu ligierenden intramuralen Arterien dient eine Ultraschalleinrichtung, welche eine am Tubus 2 angebrachte Ultraschallsonde 7 (= Ultraschallwandler) umfasst.

Das Basisteil 1 besitzt eine zentrale Durchgangsöffnung 8, deren Achse parallel zur Achse des inneren Hohlraums des Tubus 2 liegt und durch die hindurch der innere Hohlraum des Tubus 2 einsehbar ist.

Der Tubus 2 ist über eine Schnappverbindung mit dem Basisteil 1 lösbar verbunden. Ein Einsteckfortsatz 9 des Tubus 2 ist in die Durchgangsöffnung 8 des Basisteils 1 einsteckbar und dient zur Zentrierung des Tubus 2 gegenüber dem Basisteil 1. Ein Rastfortsatz 10 steht über diesen Einsteckfortsatz 9 in achsialer Richtung des Tubus 2 hinaus und besitzt an seinem Ende Rastzungen 11 mit Rastnasen 12. Der Rastfortsatz 10 ist in eine nutartige Vertiefung 13 in der Wand der Durchgangsöffnung 8 einsteckbar, wobei die Rastnasen 12 in Rastschultern 14 einschnappen. Durch einen ausreichend starken Zug am Tubus 2 kann die Verbindung wieder gelöst werden.

Das Basisteil 1 weist auf gegenüberliegenden Seiten radial abstehende Handgriffe 15, 16 auf. Durch die in Umfangsrichtung symmetrische Anordnung der Handgriffe 15, 16 wird eine bezogen auf die Drehung um die Längsachse des Tubus 2 gleichmäßige Gewichtsverteilung erreicht, wodurch die Handhabung erleichtert wird. Es können auch mehr als zwei gleichmäßig in Umfangsrichtung voneinander beabstandete Handgriffe vorhanden sein.

Das Basisteil 1 bildet ein Gehäuse 18, 19 aus, innerhalb dessen eine Ansteuer- und Auswertelektronik 20 angeordnet ist, die in Fig. nur stark schematisiert dargestellt ist, wobei einige elektronische Bauelemente angedeutet sind. Diese Ansteuer- und Auswertelektronik 20 steuert die Ultraschallsonde 7 an und wertet das von ihr empfangene reflektierte Signal aus.

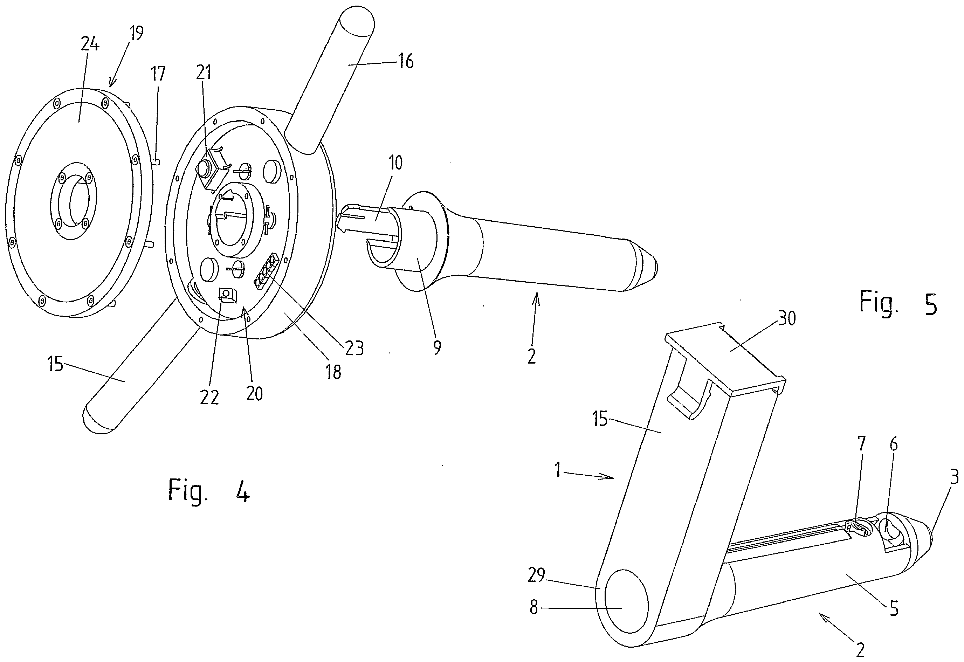

Die Ansteuer- und Auswertelektronik 20 umfasst Eingabeelemente 21, von denen in Fig. 4 nur eines schematisch dargestellt ist und die beispielsweise berührungslos durch den Gehäusedeckel 19 bedienbar ausgebildet sein können. Als Eingabeelemente 21 können beispielsweise ein Ein- und Ausschalter und ein Lautstärkeregler vorhanden sein. Die Ansteuer- und Auswertelektronik 20 umfasst weiters Ausgabeelemente 22, 23. Als Ausgabeelemente sind z. B. ein Lautsprecher (Ausgabeelement 22) und ein Leuchtdiodenbalken (Ausgabeelement 23) vorhanden. Der Lautsprecher 22 und der Leuchtdiodenbalken 23 dienen zur akustischen und optischen Anzeige der Lokalisation einer intramuralen Arterie. Der Leuchtdiodenbalken 23 ist durch einen durchsichtigen Einsatz 24 im Gehäusedeckel 19 sichtbar. Daneben können noch andere Eingabeelemente (beispielsweise zur Einstellung der Sensitivität) und/oder Ausgabeelemente (beispielsweise eine Leuchtdiode zur Anzeige des eingeschalteten Zustands und eine Leuchtdiode zur Anzeige eines niedrigen Ladezustands eines eingebauten Akkumulators).

Die Stromversorgung erfolgt durch einen Energiespeicher, in Form einer Batterie oder eines Akkumulators, der in den Fig. 1 bis 4 nicht dargestellt ist. Dieser Energiespeicher ist ebenfalls im Basisteil 1 angeordnet, beispielsweise im Gehäuseunterteil 18 oder in einem oder beiden Handgriffen 15, 16. Die Aufladung des Energiespeichers kann in herkömmlicher Weise mittels eines Ladegeräts erfolgen, berührungslos oder über Kontaktelemente.

Im gezeigten Ausführungsbeispiel ist der rückseitige Gehäusedeckel 19 über Befestigungsschrauben 17 am Gehäuseunterteil 18 angeschraubt.

Das Gehäuse 18, 19 ist insgesamt gasdicht ausgebildet, wobei miteinander verbundene Teile 18, 19 des Gehäuses entsprechend gegeneinander abgedichtet sind, beispielsweise durch zwischen den Teilen 18, 19 angeordnete elastische Dichtringe. Das Basisteil 1 ist somit insgesamt gassterilisierbar und wird für eine Mehrzahl von Operationen verwendet. Der Tubus 2 ist dagegen als Einwegteil ausgebildet und wird nach jeder Operation ersetzt.

Wenn die elektronischen Bauteile ausreichend wärmebeständig sind oder wärmegeschützt sind könnte auch eine Dampfsterilisierung des Basisteils 1 erfolgen.

Beim Einschnappen des Tubus 2 in das Basisteil 1 werden Kontaktelemente 26, 27 des Tubus 2 und des Basisteils 1 miteinander verbunden, um die Ultraschallsonde 7 mit der Ansteuer- und Auswertelektronik 20 zu verbinden.

Beim Ausführungsbeispiel gemäß den Fig. 1 bis 4 ist somit die mit der Ultraschallsonde 7 verbundene Elektronikeinheit, welche die Ansteuer- und Auswertelektronik 20 mit den Eingabeelementen 21 und Ausgabeelementen 22, 23, einen Energiespeicher und ein Gehäuse 18, 19 umfasst, ein integraler Bestandteil des Basisteils 1. Teile dieser Elektronikeinheit können auch in den Handgriffen 15, 16 des Basisteils 1 angeordnet sein.

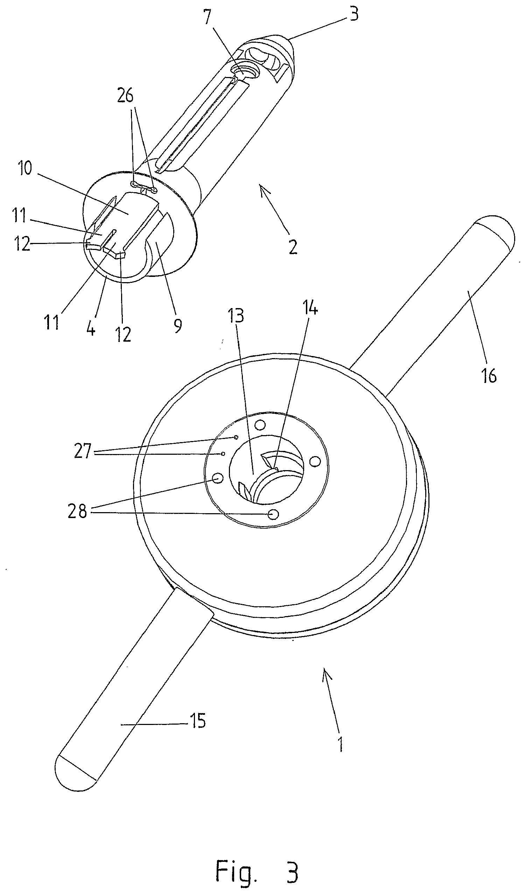

Die Elektronikeinheit umfasst auch eine Beleuchtungseinrichtung. In Fig. 3 sind Leuchtdioden 28 dieser Beleuchtungseinrichtung angedeutet, deren Licht in den Tubus 2 eingestrahlt wird. Das eingestrahlte Licht kann durch Totalreflexion bis zum distalen Bereich des Tubus 2 geleitet werden und in Richtung zur Behandlungsstelle ausgekoppelt werden, beispielsweise durch Einbringen von aufgerauten Bereichen.

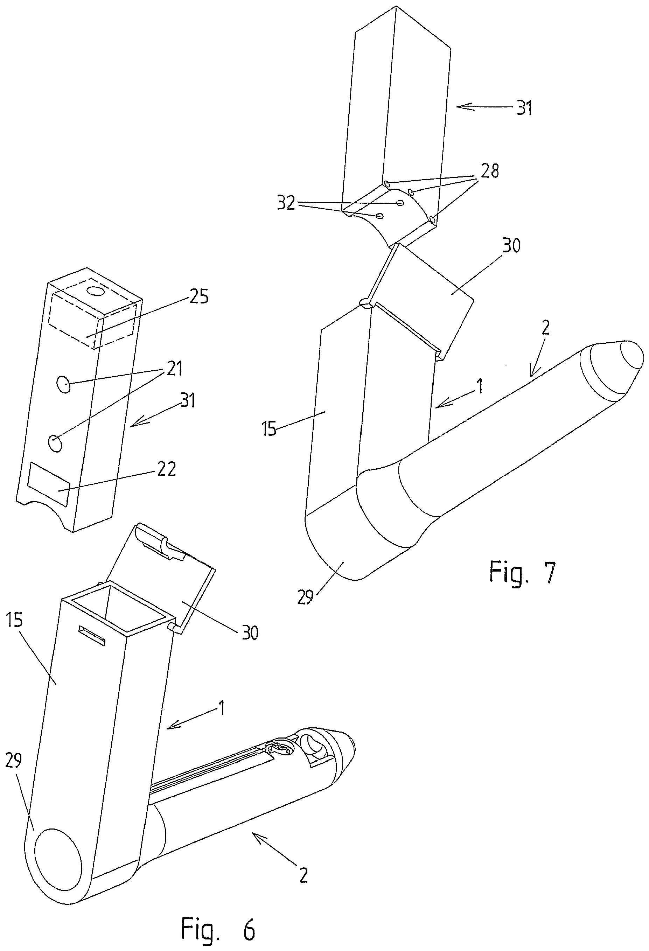

Ein Ausführungsbeispiel der Erfindung ist in den Fig. 5 bis 7 schematisch dargestellt. Der Tubus 2 ist hier mit einem Basisteil 1 starr verbunden. Das Basisteil 1 umfasst einen Verbindungsabschnitt 29 zur Verbindung des Basisteils 1 mit dem Tubus 2 und einen Handgriff 15. Der Verbindungsabschnitt 29 und der Handgriff 15 können einstückig ausgebildet sein. Auch eine einstückige Ausbildung des Basisteils 1 mit dem Tubus 2 ist denkbar und möglich. Der Handgriff 15 ist hier als öffen- und schließbares Gehäuse ausgebildet. Zu diesem Zweck besitzt er beispielsweise einen aufklappbaren endseitigen Deckel 30. In den Handgriff 15 ist eine Elektronikeinheit 31 einsetzbar. Diese umfasst die Ansteuer- und Auswertelektronik 20 mit Eingabe- und Ausgabeelementen 21, 22 und einen Energiespeicher 25, der in Fig. 6 durch strichlierte Linien angedeutet ist. Beispielsweise ist als Ausgabeelement 22 ein Bildschirm angedeutet, der durch das durchsichtige Material des Handgriffs 15 ablesbar ist. Eingabeelemente 21 können wiederum berührungslos durch das Material des Handgriffs 15 bedienbar sein. Die elektrische Verbindung der Elektronikeinheit 31 mit der Ultraschallsonde 7 erfolgt über Kontaktelemente 32 an der Elektronikeinheit 31, die beim Einsetzen in den Handgriff 15 mit entsprechenden Kontaktelementen des Basisteils 1 in Berührung kommen.

An der Elektronikeinheit 31 sind weiters Leuchtdioden 28 angeordnet, die Licht in den Tubus 2 zur Beleuchtung der Behandlungsstelle einstrahlen.

Das Basisteil 1 und der darin angebrachte Tubus 2 sind bei dieser Ausführungsform als Einmalteile ausgebildet. Die Elektronikeinheit 31 muss nicht sterilisiert werden und ist mehrfach verwendbar.

Anstelle von berührungslosen Eingabeelementen 21 können auch Folientasten im Handgriff 15 (Fig. 5 bis 7) bzw. im Gehäusedeckel 19 (Fig. 1 bis 4) eingesetzt werden.

Insgesamt ist die Einrichtung somit wie beschrieben als autarkes Handgerät ausgebildet, welches während der Operation keine weiteren Komponenten benötigt. Durch den Wegfall eines Verbindungskabels wird auch die Einstrahlung von Störsignalen minimiert. Durch den im Basisteil 1 angeordneten Lautsprecher wird die akustische Überwachung für den Operateur erleichtert. Durch die im Basisteil 1 angeordneten Eingabeelemente (Bedienelemente) wird die Bedienbarkeit verbessert, beispielsweise die Lautstärkeregelung.

Eine erfindungsgemäße Einrichtung kann in der Weise ausgebildet sein, dass für die Ligierung der Arterie mittels der Ultraschallsonde erfasste relevante Informationen, wie die Lage und die Tiefe (unterhalb der Mukosa) der zu ligierenden Arterie und die Größe der Arterie, durch eine optische Anzeige im direkten Blickfeld des Operateurs an der dem Operateur zugewandten Stirnseite der Einrichtung dargestellt werden können. Der Operateur muss somit zum Erhalt dieser Informationen den Kopf nicht seitwärts vom Operationsbereich wegdrehen, wodurch eine größere Operationssicherheit erreicht wird.

Unterschiedliche weitere Modifikationen des gezeigten Ausführungsbeispiels sind denkbar und möglich, ohne den Bereich der Erfindung zu verlassen. So könnte beispielsweise ein Basisteil, welches ein Gehäuse zur Aufnahme einer separaten einsetzbaren und herausnehmbaren Elektronikeinheit ausbildet, eine Form analog der in den Fig. 1 bis 4 dargestellten Ausführungsform aufweisen. Ein Gehäusedeckel könnte beispielsweise mit einem Bajonettverschluss mit einem Gehäuseunterteil verbunden sein, sodass das Gehäuse leicht geöffnet werden kann. Das Basisteil 1 und der Tubus 2, der in diesem Fall starr mit dem Basisteil verbunden sein kann, könnten hierbei als Einwegteile ausgebildet sein.

Die beim Ausführungsbeispiel gemäß den Fig. 1 bis 4 am Gehäuseunterteil 18 angeordneten Handgriffe 15, 16 könnten auch entfallen und das Gehäuseunterteil beispielsweise an seinem äußeren Umfang gerändelt ausgebildet sein und selbst den Handgriff bilden. Auch eine Ausbildung in der Form eines Speichenrades wäre denkbar und möglich, wobei der Handgriff eine Ringform besitzt und hierbei konzentrisch zur Längsachse des Tubus 2 liegt.

Als Ausgabeelement könnte beispielsweise auch ein Vibrationsalarm vorgesehen sein, der die Lokalisation einer Arterie anzeigt.

**Legende zu den Hinweisziffern:**

| | | | |
|---|---|---|---|
| 1 | Basisteil | 21 | Eingabeelement |
| 2 | Tubus | 22 | Ausgabeelement |
| 3 | distales Ende | 23 | Ausgabeelement |
| 4 | proximales Ende | 24 | Einsatz |
| 5 | Mantelwand | 25 | Energiespeicher |
| 6 | Behandlungsöffnung | 26 | Kontaktelement |
| 7 | Ultraschallsonde | 27 | Kontaktelement |
| 8 | Durchgangsöffnung | 28 | Leuchtdiode |
| 9 | Einsteckfortsatz | 29 | Verbindungsabschnitt |
| 10 | Rastfortsatz | 30 | Deckel |
| 11 | Rastzunge | 31 | Elektronikeinheit |
| 12 | Rastnase | 32 | Kontaktelement |
| 13 | Vertiefung | | |
| 14 | Rastschulter | | |
| 15 | Handgriff | | |
| 16 | Handgriff | | |
| 17 | Befestigungsschraube | | |
| 18 | Gehäuseunterteil | | |
| 19 | Gehäusedeckel | | |
| 20 | Ansteuer- und Auswertelektronik | | |

## Patentansprüche

1. Einrichtung zur Verwendung bei der Ligatur von intramuralen Arterien in Hohlorganen, mit einem mindestens einen Handgriff (15, 16) aufweisenden Basisteil (1) und einem am Basisteil (1) angebrachten, in das Hohlorgan, insbesondere das Rektum, einzuführenden Tubus (2), an dem zur Ortung der zu legierenden Arterien eine Ultraschallsonde (7) angeordnet ist, die mit einer Ansteuer- und Auswertelektronik (20) verbunden ist, welche Ein- und Ausgabeelemente (21, 22, 23) umfasst, **dadurch gekennzeichnet, dass** die Ansteuer- und Auswertelektronik (20) und ein Energiespeicher (25) zur Stromversorgung der Ansteuer- und Auswertelektronik (20) im Basisteil (1) angeordnet sind, wobei das Basisteil (1) ein Gehäuse aufweist, innerhalb dessen eine mit einem eigenen Gehäuse versehene Elektronikeinheit (31) angeordnet ist, welche die Ansteuer- und Auswertelektronik (20) mit den Ein- und Ausgabeelementen (21, 22, 23) und den Energiespeicher (25) umfasst, und das Gehäuse des Basisteils (1) öffen- und schließbar ist, wobei im geöffneten Zustand des Gehäuses des Basisteils (1) die Elektronikeinheit (31) herausnehmbar und einsetzbar ist.

2. Einrichtung nach Anspruch 1, **dadurch gekennzeichnet, dass** der Tubus (2) mit der an ihm angeordneten Ultraschallsonde (7) vom Basisteil (1) abnehmbar ausgebildet ist.

3. Einrichtung nach Anspruch 2, **dadurch gekennzeichnet, dass** der Tubus (2) mit dem Basisteil (1) über eine Schnappverbindung verbunden ist.

4. Einrichtung nach einem der Ansprüche 1 bis 3, **dadurch gekennzeichnet, dass** auf der vom Tubus (2) abgewandten Seite des Basisteils (1) Ein- und Ausgabeelemente (21, 22, 23) der Ansteuer- und Auswertelektronik (20) angeordnet sind.

5. Einrichtung nach einem der Ansprüche 1 bis 4, **dadurch gekennzeichnet, dass** das Basisteil (1) mindestens zwei in Umfangsrichtung gleichmäßig voneinander beabstandete Handgriffe (15, 16) aufweist.

6. Einrichtung nach einem der Ansprüche 1 bis 4, **dadurch gekennzeichnet, dass** das Basisteil (1) einen zur Längsachse des Tubus (2) konzentrischen ringförmigen Handgriff aufweist.

7. Einrichtung nach einem der Ansprüche 1 bis 6, **dadurch gekennzeichnet, dass** das Basisteil (1) eine Durchgangsöffnung (8) aufweist, durch die hindurch der innere Hohlraum des Tubus (2) einsehbar ist.

8. Einrichtung nach einem der Ansprüche 1 bis 7, **dadurch gekennzeichnet, dass** ein Ausgabeelement (22) der Ansteuer- und Auswertelektronik (20) ein Lautsprecher ist.

9. Einrichtung nach einem der Ansprüche 1 bis 8, **dadurch gekennzeichnet, dass** ein Ausgabeelement (23) der Ansteuer- und Auswertelektronik (20) ein Bildschirm ist.

10. Einrichtung nach einem der Ansprüche 1 bis 9, **dadurch gekennzeichnet, dass** ein Eingabeelement (21) der Ansteuer- und Auswertelektronik (20) ein Lautstärkeregler ist.

## Claims

1. Device for use in the ligation of intramural arteries in hollow organs, having a base part (1) which has at least one handle (15, 16) and having, mounted on the base part (1), a tube (2), for insertion in the hollow organ and in particular in the rectum, on which is arranged an ultrasonic probe (7) for locating the arteries to be ligated, which ultrasonic probe (7) is connected to driving and analysing electronics (20) which comprise input and output members (21, 22, 23), **characterised in that** the driving and analysing electronics (20) and an energy-storing means (25) for supplying power to the driving and analysing electronics (20) are arranged in the base part (1), the base part (1) having a shell within which is arranged an electronics unit (31) which is provided with a shell of its own and which comprises the energy-storing means (25) and the driving and analysing electronics (20) having the input and output members (21, 22, 23), and the shell of the base part (1) can be opened and closed, the electronics unit (31) being removable and insertable when the shell of the base part (1) is in the open state.

2. Device according to claim 1, **characterised in that** the tube (2), together with the ultrasonic probe (7) arranged thereon, is formed to be detachable from the base part (1).

3. Device according to claim 2, **characterised in that** the tube (2) is connected to the base part (1) by a snap-in connection.

4. Device according to one of claims 1 to 3, **characterised in that** input and output members (21, 22, 23) of the driving and analysing electronics (20) are arranged on the side of the base part (1) remote from the tube (2).

5. Device according to one of claims 1 to 4, **characterised in that** the base part (1) has at least two handles (15, 16) which are evenly spaced apart from one another in the circumferential direction.

6. Device according to one of claims 1 to 4, **characterised in that** the base part (1) has an annular handle concentric with the longitudinal axis of the tube (2) .

7. Device according to one of claims 1 to 6, **characterised in that** the base part (1) has a through-opening (8) through which the inner cavity in the tube (2) can be seen into.

8. Device according to one of claims 1 to 7, **characterised in that** an output member (22) of the driving and analysing electronics (20) is a loudspeaker.

9. Device according to one of claims 1 to 8, **characterised in that** an output member (23) of the driving and analysing electronics (20) is a display screen.

10. Device according to one of claims 1 to 9, **characterised in that** an input member (21) of the driving and analysing electronics (20) is a volume control.

## Revendications

1. Dispositif destiné à être utilisé pour la ligature d'artères intramurales dans des organes creux, comprenant une partie de base (1) comportant au moins une poignée (15, 16) ainsi qu'un tube (2) monté sur la partie de base (1) destiné à être introduit dans l'organe creux, en particulier le rectum, et sur lequel est monté, pour permettre la localisation de l'artère à ligaturer, une sonde à ultrasons (7) qui est reliée à une électronique de commande et d'exploitation (20) comportant des éléments d'entrée et de sortie (21, 22, 23),
**caractérisé en ce que**
l'électronique de commande et d'exploitation (20) et un accumulateur d'énergie (25) permettant d'alimenter l'électronique de commande et d'exploitation (20) en courant sont montés dans la partie de base (1), la partie de base (1) comportant un boîtier à la partie interne duquel est montée une unité électronique (31) équipée d'un boîtier propre, qui comporte l'électronique de commande et d'exploitation (20) avec les éléments d'entrée et de sortie (21, 22, 23) et l'accumulateur d'énergie (25), le boîtier de la partie de base (1) pouvant être ouvert ou fermé, et, à l'état ouvert du boîtier de la partie de base (1), l'unité électronique (31) pouvant être extraite et insérée.

2. Dispositif conforme à la revendication 1,
**caractérisé en ce que**
le tube (2) et la sonde à ultrasons (7) montée sur celui-ci peuvent être extraits de la partie de base (1).

3. Dispositif conforme à la revendication 2,
**caractérisé en ce que**
le tube (2) est relié à la partie de base (1) par l'intermédiaire d'une liaison par encliquetage.

4. Dispositif conforme à l'une des revendications 1 à 3,
**caractérisé en ce que**
sur le côté de la partie de base (1) situé à l'opposé du tube (2) sont montés des éléments d'entrée et de sortie (21, 22, 23) de l'électronique de commande et d'exploitation (20).

5. Dispositif conforme à l'une des revendications 1 à 4,
**caractérisé en ce que**
la partie de base (1) comporte au moins deux poignées (15, 16) équidistantes dans la direction périphérique.

6. Dispositif conforme à l'une des revendications 1 à 4,
**caractérisé en ce que**
la partie de base (1) comporte une poignée annulaire concentrique à l'axe longitudinal du tube (2).

7. Dispositif conforme à l'une des revendications 1 à 6,
**caractérisé en ce que**
la partie de base (1) comporte une ouverture traversante (8) au travers de laquelle peut être observé le volume creux interne du tube (2).

8. Dispositif conforme à l'une des revendications 1 à 7,
**caractérisé en ce qu'**
un élément de sortie (22) de l'électronique de commande et d'exploitation (20) est un haut-parleur.

9. Dispositif conforme à l'une des revendications 1 à 8,
**caractérisé en ce qu'**
un élément de sortie (23) de l'électronique de commande et d'exploitation (20) est un écran.

10. Dispositif conforme à l'une des revendications 1 à 9,
**caractérisé en ce qu'**
un élément d'entrée (21) de l'électronique de commande et d'exploitation (20) est un régulateur d'intensité sonore.
